# EUROPÄISCHE PATENTSCHRIFT

(11) **EP 1 317 415 B1**
(45) Veröffentlichungstag und Bekanntmachung des Hinweises auf die Patenterteilung: **23.03.2005**
(21) Anmeldenummer: 01985698.8
(22) Anmeldetag: 30.08.2001
(51) Int. Cl.: C07C 241/00, C07C 243/02

(54) **VERFAHREN ZUR HERSTELLUNG VON DNDA**
METHOD FOR PRODUCING DNDA
PROCEDE DE PRODUCTION DE DNDA

(30) Priorität: 30.08.2000 DE 10042862
(43) Veröffentlichungstag der Anmeldung: 11.06.2003
(73) Patentinhaber: DynITEC GmbH, 53840 Troisdorf (DE)
(72) Erfinder: SCHIRRA, Rainer, 53797 Lohmar (DE); EMANS, Heinz-Gerd, 53859 Niederkassel (DE); LICHTBLAU, Leonard, 51147 Köln (DE)
(74) Vertreter: Uppena, Franz, Dr.
(86) Internationale Anmeldenummer: PCT/EP2001/010023
(87) Internationale Veröffentlichungsnummer: WO 2002/026692

(56) Entgegenhaltungen:
- WO-A-01/46129
- WO-A-01/64627
- GB-A- 1 111 707
- US-A- 4 476 322
- US-A- 4 513 148
- DATABASE WPI Section Ch, Week 200062 Derwent Publications Ltd., London, GB; Class A60, AN 2000-645688 XP002196939 & RU 2 148 574 C (ZELINSKII ORGANIC CHEM INST), 10. Mai 2000 (2000-05-10)
- TARTAKOVSKII, V.. A. ET AL.: "Preparation of N, N'-dialkyl methylenebisnitramines" RUSSIAN CHEMICAL BULLETIN, Bd. 42, Nr. 11, 1993, Seiten 1916-1918, XP001073453
- 'RÖMPP CHEMIE LEXIKON', 1992, GEORG THIEME VERLAG * Seite 3973 - Seite 3974 *

## Beschreibung

Die Erfindung betrifft ein Verfahren zur Herstellung von DNDA.

Bisher wurde der energetische Weichmacher DNDA-5-7, ein ternäres Gemisch aus N,N'-Dimethylbisnitramin (DNDA-5; ca. 43%), N-Methyl-N'-Ethyl-bisnitramin (DNDA-6; ca. 45%) und N,N'-Diethylbisnitramin (DNDA-7; ca. 12%), auf nachfolgende Weise hergestellt:
1. Nitrierung von N,N'-Dimethylharnstoff
   1.1. Batchreaktion mit Mischsäure (Salpetersäure-Schwefelsäuregemisch) unter Eintrag des Harnstoffes gelöst in Dichlormethan bei ca. -10°C
   1.2. Abbruch der Reaktion durch Ausgießen auf Eiswasser
   1.3. Aufnehmen des Reaktionsgemisches (Hauptprodukt: Dinitrodimethylharnstoff) in Dichlormethan durch Ausschütteln
   1.4. Neutralisieren der Dichlormethanphase durch Natriumhydrogencarbonat
2. Hydrolyse des Dinitrodimethylharnstoffes
   2.1. Mischen der Dichlormethanphase mit Wasser, Abdampfen des Dichlormethans und anschließende Hydrolyse bei ca. 100°C
   2.2. Aufnehmen des Methylnitramins in Dichlormethan
3. Nitrierung von N,N'-Diethylharnstoff
   3.1. Batchreaktion mit Mischsäure (Salpetersäure-Schwefelsäuregemisch) unter Eintrag des Harnstoffes gelöst in Dichlormethan bei ca. -15°C
   3.2. Abbruch der Reaktion durch Ausgießen auf Eiswasser
   3.3. Aufnehmen des Reaktionsgemisches (Hauptprodukt: Dinitrodiethylharnstoff) in Dichlormethan durch Ausschütteln
   3.4. Neutralisieren der Dichlormethanphase durch Natriumhydrogencarbonat
4. Hydrolyse des Dinitrodiethylharnstoffes
   4.1. Mischen der Dichlormethanphase mit Wasser, Abdampfen des Dichlormethans und anschließende Hydrolyse bei ca. 100°C
   4.2. Aufnehmen des Ethylnitramins in Dichlormethan
5. Kondensation
   5.1. Gemeinsame Kondensation des Methyl- und Ethylnitramins in einem festgelegten Verhältnis von ca. 2:1 bei max. 0°C mit 70%iger Schwefelsäure und p-Formaldehyd in Dichlormethan
   5.2. Abbruch der Reaktion durch Ausgießen auf Eiswasser
   5.3.Aufnehmen des Reaktionsgemisches (ternäres Gemisch der Nitramine) in Dichlormethan durch Ausschütteln
   5.4. Neutralisieren der Dichlormethanphase durch Natriumhydrogencarbonat

Das oben skizzierte Verfahren ist nicht geeignet, größere Mengen des Weichmachers in ökonomisch sinnvoller Weise herzustellen.

Aufgabe der vorliegenden Erfindung war es daher, ein Verfahren zur Herstellung des Weichmachers vom Typ DNDA, insbesondere ein Verfahren zur Herstellung von DNDA-5-7 bereitzustellen.

Gelöst wurde diese Aufgabe durch ein Verfahren, bei dem nachfolgende Änderungen des aus dem Stand der Technik bekannten Verfahrens vorgenommen wurden:
- Anstelle der Mischsäure wird hochkonzentrierte Salpetersäure in deutlichem vorzugsweise etwa 11-fachem molaren Überschuss eingesetzt. Diese Änderung ermöglicht die Recyclierung der Salpetersäure, außerdem entstehen überraschenderweise weniger Nebenprodukte.
- Die Harnstoffe werden nicht in Dichlormethan gelöst, sondern als Feststoffe eingetragen. Die Vorteile dieser Variante: geringeres Flüssigkeitsvolumen, kein Verdünnungseffekt.
- N,N'-Dimethyl- und N,N'-Diethylharnstoff werden nicht getrennt nitriert, sondern gemeinsam bei max. 5°C in dem gewünschten molaren Verhältnis, vorzugsweise einem molaren Verhältnis von etwa 2:1 (DNDA-5-7). Der Vorteil besteht darin, dass Prozessschritte eingespart werden können.
- Das Reaktionsgemisch (Hauptprodukte: Dinitrodimethyl- und Dinitrodiethylharnstoff) wird in Dichlormethan durch Gegenstromextraktion aufgenommen. Hierdurch werden Prozessschritte eingespart und die Trennleistung optimiert.
- Gemeinsames Hydrolysieren des so erhaltenen Gemisches bei etwa 100°C im gegebenem molaren Verhältnis. Vorteil: Einsparen von Prozessschritten.
- Die gesamte Reaktion wird kontinuierlich durchgeführt. Dadurch ist es möglich, die Reaktionswärme besser abzuführen, die Reaktionsvolumina werden reduziert, wodurch sich das Gefahrenpotential verringert.

Mit den erfindungsgemäßen Änderungen des aus dem Stand der Technik bekannten Verfahrens ist es möglich, DNDA in hoher Ausbeute, in hoher Reinheit und in der gewünschten Zusammensetzung unmittelbar herzustellen.

Mittels HPLC-Untersuchungen wurde überraschenderweise festgestellt, dass sowohl die gewünschten Produkte, wie auch die gewünschten Zusammensetzungen entstehen und zusätzliche Nebenprodukte bei dieser Art der erfindungsgemäßen Prozessführung nicht entstehen.

Es wird ausdrücklich darauf hingewiesen, dass es nicht erforderlich ist, alle Verfahrensschritte in der erfindungsgemäßen Weise durchzuführen. Eine Verbesserung des im Stand der Technik beschriebenen Verfahrens ist bereits zu erzielen, wenn mindestens einer der durchgeführt wird. Eine besonders bevorzugte Verfahrensführung ist jedoch die Kombination aller erfindungsgemäßen Varianten.

Die nachfolgenden Beispiele sollen die Erfindung näher erläutern ohne sie einzuschränken:

### Gemeinsame Nitrierung der Harnstoffe:

355 ml HNO₃ (100%) wurden auf -15°C runtergekühlt. Ein Gemisch aus 25 g Diethylharnstoff und 50g Dimethylharnstoff wurde portionsweise unter starkem Rühren zugegeben. Hierbei sollte die Temperatur -10°C nicht übersteigen. Die Rührzeit nach beendeter Zugabe betrug noch 30 min. Die Reaktionslösung wurde dann auf Eiswasser geleitet und in jeweils 250 ml Methylenchlorid durch mehrfaches, vorzugsweises vierfaches Ausschütteln aufgenommen. Neutralisieren erfolgte durch eine wässrige Bicarbonat-Lösung.

### Gemeinsame Hydrolyse der Dinitrodimethyl- und Dinitodiethylharnstoffe:

Ein Gemisch aus 1000 ml der obigen Lösung und 540 ml Wasser wurde im Ölbad erhitzt. Hierbei erfolgte zunächst bei Sumpftemperaturen von ca. 50°C Abdampfen der Methylenchloridphase. Nach Umstellen auf Rückflußkühlung wurde das Gemisch über einen Zeitraum von mehreren Stunden, vorzugsweise über einen Zeitraum von etwa 3 h, bei einer Sumpftemperatur von etwa 96°C hydrolysiert. Die Abtrennung des Nitramin-Gemisches erfolgte durch mehrfaches, vorzugsweise dreifaches Ausschütteln mit jeweils etwa 500 ml Dichlormethan. Durch Abdampfen im Rotationsverdampfer wurde die Nitraminlösung auf etwa 95 ml eingeengt.

### Kondensation:

Zu einer Vorlage von 120 ml Schwefelsäure (75%) erfolgte bei 0°C die Zugabe von 5 g Paraformaldehyd, welches in 40 ml Methylenchlorid gelöst war. Die 95 ml Nitraminlösung aus dem vorherigen Schritt wurden aus einem Tropftrichter in 30 Minuten unter Rühren zudosiert. Um die Konzentration der Schwefelsäure in etwa konstant zu halten, und um die Ausbeute zu optimieren, wurde zusätzlich 96 %ige Schwefelsäure zugeführt. Die Temperatur sollte hierbei vorzugsweise nicht über 0°C steigen. Die Kondensation war nach etwa 3 h Rührzeit bei 0°C abgeschlossen. Die Reaktion wurde durch Ausgießen auf Eiswasser beendet und das Produkt erneut in Methylenchlorid aufgenommen, neutralisiert, gewaschen und eingeengt. Ausbeute: 35 g DNDA-5-7.

Mit dem erfindungsgemäßen Verfahren ist es damit erstmals möglich, DNDA ökonomisch, chemisch und verfahrenstechnisch sinnvoll in hoher Ausbeute, hoher Reinheit und in der gewünschten Zusammensetzung herzustellen.

## Patentansprüche

1. Verfahren zur Herstellung von DNDA durch Nitrierung von N,N'-Dimethylharnstoff und N,N'-Diethylharnstoff, Hydrolyse der Dinitrodimethylharnstoffes und des Dinitrodiethylharnstoffes und Kondensation des Methyl- und Ethylnitramins, **dadurch gekennzeichnet, dass** zur Nitrierung der Harnstoffe 100 %ige Salpetersäure eingesetzt wird.

2. Verfahren gemäß Anspruch 1, **dadurch gekennzeichnet, dass** die Harnstoffe als Feststoff eingesetzt werden.

3. Verfahren gemäß Anspruch 1 oder 2, **dadurch gekennzeichnet, dass** N,N'-Dimethyl- und N,N'-Diethylharnstoff gemeinsam nitriert werden.

4. Verfahren gemäß einem oder mehreren der Ansprüche 1 bis 3, **dadurch gekennzeichnet, dass** das Reaktionsgemisch aus der Nitrierung (Hauptprodukte: Dinitrodimethyl- und Dinitrodiethylharnstoff) in Dichlormethan durch Gegenstromextraktion aufgenommen wird.

5. Verfahren gemäß einem oder mehreren der Ansprüche 1 bis 4, **dadurch gekennzeichnet, dass** Dinitrodimethylharnstoff und Dinitrodiethylharnstoff gemeinsam hydrolysiert werden.

6. Verfahren gemäß Anspruch 5, **dadurch gekennzeichnet, dass** Dinitrodimethylharnstoff und Dinitrodiethylharnstoff bei 100°C im gewünschten molaren Verhältnis gemeinsam hydrolysiert wird.

7. Verfahren zur Herstellung von DNDA-5-7 durch Nitrierung von N,N'-Dimethylharnstoff und N,N'-Diethylharnstoff, Hydrolyse der Dinitrodimethylharnstoffes und des Dinitrodiethylharnstoffes und Kondensation des Methyl- und Ethylnitramins, **dadurch gekennzeichnet, dass** zur Nitrierung der Harnstoffe 100 %ige Salpetersäure eingesetzt wird, dass die Harnstoffe als Feststoffe eingesetzt werden, dass N,N'-Dimethyl- und N,N'-Diethylharnstoff im molaren Verhältnis von 2:1 gemeinsam nitriert werden und dass Dinitrodimethylharnstoff und Dinitrodiethylharnstoff gemeinsam hydrolysiert werden.

8. Verfahren gemäß einem oder mehreren der Ansprüche 1 bis 8, **dadurch gekennzeichnet, dass** die Verfahrensschritte kontinuierlich durchgeführt werden.

9. Verwendung des gemäß einem der vorhergehenden Ansprüche hergestellten Weichmachers DNDA in Treibladungspulver.

## Claims

1. Method for producing DNDA by nitration of N,N'-dimethylurea and N,N'-diethylurea, hydrolysis of the dinitrodimethylurea and the dinitrodiethylurea and condensation of the methyl nitramine and ethyl nitramine, **characterised in that** 100% nitric acid is used for the nitration of the ureas.

2. Method according to claim 1, **characterised in that** the ureas are used as solid matter.

3. Method according to claim 1 or 2, **characterised in that** N,N'-dimethylurea and N,N'-diethylurea are nitrated together.

4. Method according to one or more of claims 1 to 3, **characterised in that** the reaction mixture from the nitration (main products: dinitrodimethylurea and dinitrodiethylurea) are taken up in dichloromethane by counterflow extraction.

5. Method according to one or more of claims 1 to 4, **characterised in that** dinitrodimethylurea and dinitrodiethylurea are hydrolyzed together.

6. Method according to claim 5, **characterised in that** dinitrodimethylurea and dinitrodiethylurea are hydrolyzed together at 100°C in the desired molar ratio.

7. Method for producing DNDA-5-7 by nitration of N,N'-dimethylurea and N,N'-diethylurea, hydrolysis of the dinitrodimethylurea and the dinitrodiethylurea and condensation of the methyl nitramine and ethyl nitramine, **characterised in that** 100% nitric acid is used for the nitration of the ureas, **in that** the ureas are used as solids, **in that** N,N'-dimethylurea and N,N'-diethylurea are nitrated together in a molar ratio of 2:1, and **in that** dinitrodimethylurea and dinitrodiethylurea are hydrolyzed together.

8. Method according to one or more of claims 1 to 8 *(sic)*, **characterised in that** the method steps are carried out continuously.

9. Use of the plasticizer DNDA, produced in accordance with one of the preceding claims, in propellant charge powder.

## Revendications

1. Procédé de préparation de DNDA par nitration de N,N'-diméthyl-urée et de N,N'-diéthyl-urée, hydrolyse de la dinitro-diméthyl-urée et de la dinitro-diéthyl-urée, et condensation de la méthyl-nitramine et de l'éthyl-nitramine, **caractérisé en ce que**, pour la nitration des urées, on emploie de l'acide nitrique à 100 %.

2. Procédé conforme à la revendication 1, **caractérisé en ce que** les urées sont employées à l'état solide.

3. Procédé conforme à la revendication 1 ou 2, **caractérisé en ce que** la N,N'-diméthyl-urée et la N,N'-diéthyl-urée sont nitrées ensemble.

4. Procédé conforme à l'une ou plusieurs des revendications 1 à 3, **caractérisé en ce que** le mélange réactionnel issu de la nitration, dont les produits principaux sont de la dinitro-diméthyl-urée et de la dinitro-diéthyl-urée, est traité par extraction à contre-courant avec du dichlorométhane.

5. Procédé conforme à l'une ou plusieurs des revendications 1 à 4, **caractérisé en ce que** la dinitro-diméthyl-urée et la dinitro-diéthyl-urée sont hydrolysées ensemble.

6. Procédé conforme à la revendication 5, **caractérisé en ce que** la dinitro-diméthyl-urée et la dinitro-diéthyl-urée sont hydrolysées ensemble à 100 °C, en le rapport molaire voulu.

7. Procédé de préparation de DNDA-5-7 par nitration de N,N'-diméthyl-urée et de N,N'-diéthyl-urée, hydrolyse de la dinitro-diméthyl-urée et de la dinitro-diéthyl-urée, et condensation de la méthyl-nitramine et de l'éthyl-nitramine, **caractérisé en ce que**, pour la nitration des urées, on emploie de l'acide nitrique à 100 %, **en ce que** les urées sont employées à l'état solide, **en ce que** la N,N'-diméthyl-urée et la N,N'-diéthyl-urée sont nitrées ensemble en un rapport molaire de 2/1, et **en ce que** la dinitro-diméthyl-urée et la dinitro-diéthyl-urée sont hydrolysées ensemble.

8. Procédé conforme à l'une ou plusieurs des revendications 1 à 7, **caractérisé en ce que** les étapes du procédé sont effectuées en mode continu.

9. Emploi d'un plastifiant DNDA préparé conformément à l'une des revendications précédentes dans une poudre pour charge propulsive.
